# EUROPEAN PATENT APPLICATION

(11) **EP 3 061 501 A1**
(43) Date of publication of application: **31.08.2016**
(21) Application number: 15000576.7
(22) Date of filing: 27.02.2015
(51) Int. Cl.: A61Q 19/00, A61K 8/34, A61K 8/365, A61K 8/97, A61K 36/76, A61K 8/60

(54) **Composition for the treatment of acne**

(71) Applicant: Rottapharm Ltd., Dublin 15 (IE)
(72) Inventor: Zanardi, Andrea, 20156 Milano (MI) (IT); Gasparri, Franco, 40020 Casalfiumanese (BO) (IT); Mantegazza, Raffaella, 20900 Monza (MB) (IT); Beltrandi, Martina, 12084 Mondovì (IT)
(74) Representative: Küch, Angelika

(57) **Abstract**

The present invention describes a cosmetic or dermatological composition comprising a combination of vegetal extracts titrated in salicin and 1,2-alkanediols, preferably 1, 2-decanediol and willow bark (*Salix alba*) extract, useful for the treatment of acne.

## Description

The present invention describes a cosmetic or dermatological composition comprising a combination of vegetal extracts titrated in salicin and 1,2-alkanediols, preferably 1, 2-decanediol and willow bark (*Salix alba*) extract, useful for the treatment of acne. Acne vulgaris is the most frequently diagnosed dermatosis in patients in the age between 11 and 30. It is believed that acne affects about 80% of persons in this age group or even, taking into account lesions of low intensity, 100% of young people. The aetiopathogenesis of acne is multifactorial. In all patients with acne, the following symptoms occur: excessive sebum production, excessive keratosis of excretory ducts and openings of sebaceous glands, development of bacterial flora and release of inflammatory mediators in the skin.

In 95% of the patients, lesions are situated on the face and on the upper parts of the trunk, occasionally on the other parts of the body, and due to this location and the chronic nature of the disease, the patients have serious psychological problems.

Excessive sebum production and accumulation and blocked sebaceous gland openings favour bacterial colonization. Microcomedones are inhabited mostly by *Propionibacterium acnes,* a micro-organism mainly involved in the development of acne. Other bacteria are inter alia: *Staphylococcus epidermidis* and *Malasezzia furfur.* Due to the presence of lipase, this microorganism hydrolyses sebum di- and triglycerides to free fatty acids. Free fatty acids, which arise during the hydrolysis process, have the irritating, proinflammatory effect and intensify follicular keratosis. Also hyaluronidase, proteases and neuraminidases, produced by *Propionibacterium acnes,* have the pro-inflammatory effect. Moreover, this microorganism releases low-molecular chemotactic factors (peptides), attracting neutrocytes, and it activates both the alternative complement pathway and a classic immune response. The activator of the alternative complement pathway is the cell wall of *Propionibacterium* (B. Bergler-Czop, International Journal of Cosmetic Science, 2014, 36, 187-194).

Microorganisms like *P. acnes* can prompt the secretion of a variety of cutaneous antimicrobial peptides (AMPs). The inflammatory effects of *P. acnes* do not require the organism to be alive or be present in its entirety. In vitro, supernatants containing either membranous peptidoglycans (PG) and lipopolysaccharides (LPS) or, cytosolic contents or membrane proteins were able to increase keratinocyte expression of TLR2 and TLR4.

The extract of white willow bark (*Salix alba*) contains salicin and its derivatives, which have been used for longer than a century as antipyretic agents and in the treatment of many rheumatic disorders.

Herbal medicinal products such as various formulations from the bark of Salix species are well recognised throughout Europe for treating inflammatory conditions and pains of different origin (ESCOP, 1997).

*Salix* extracts have demonstrated analgesic, antiphlogistic and antipyretic effects in various animal models (ESCOP, 1997; Leslie, 1978; Loniewski et al. 2002).

For example, dose-dependent effects have been reported in the hot-plate and carrageenan-induced paw edema tests in rats (established test procedures to demonstrate analgesic and anti-inflammatory effects, respectively) for a *Salix* extract containing 12% salicin (Loniewski et al. 2002).

Salicin has been known as a potent anti-inflammatory agent when taken orally.

It is believed that salicin may have anti-aging capabilities when applied topically to human skin. The data from a study show that topical application of a serum formulation containing 0.5% salicin offers wide-spectrum anti-aging benefits, targeting the major signs of visible human skin aging. Statistical improvements were indicated in structure-related signs of skin aging against baseline. These include wrinkles, fine lines, jaw-line contour, firmness, extensibility, and density. Statistical improvements were also seen in mottled pigmentation, uneven skin tone, radiance, hydration and tactile roughness against baseline. These results substantiate salicin's ability to improve the visible signs of aging,
when applied topically to human skin in a product formulation. (Gopaul Remona et al., An evaluation of the effect of a topical product containing salicin on the visible signs of human skin aging Journal of Cosmetic Dermatology, 9, 196-201).

From prior art it has been known that salicin has may be useful as anti-irritative active compound in cosmetic and topical dermatological preparations (EP 0801946).

Salicin is also useful in combinations with additional herbal extracts. DE 10034328 discloses a cosmetic formulation for external application to skin, especially skin reddened by acne rosaceae, containing quercetin, rutin, salicin and escin in aqueous medium.

1,2-Alkanediols like 1,2-pentanediol (INCI: Pentylene Glycol), 1,2-hexane diol (INCI: 1,2-Hexanediol) and 1,2-octanediol (INCI: Caprylyl Glycol) as well as combinations thereof are multifunctional ingredients widely used in personal care products as moisturizers and antibacterial agents. It is well known to the person skilled in the art that these substances show specific antibacterial activity against *P. acnes.*

WO 2011117126 describes a combination of 1,2-decanediol and licochalcone A and carnitine which is effective against *Propionibacterium acnes* (*P*. *acnes*) without irritating the skin.

EP 1731036 discloses mixtures containing different 1,2-alkanediols for acne treatment.

Conventional products for the treatment of oily and blemished skin, eg. aqueous/ethanolic and / or surfactant-containing cleaning products have the disadvantage to strain usually the skin to dry out and work only little skin caring. It is particularly disadvantageous that the body's reaction to the use of ethanol and / or detergents-containing solutions over a longer period of time may be an overproduction of sebum, which counteracts the primary therapeutic goal of sebum reduction in oily, blemished and acne prone skin.

For the topical treatment of acne there are acne agents, such as strong oxidizing agents, such as benzoyl peroxide; alpha-hydroxy acids, such as salicylic acid and lactic acid; aliphatic dicarboxylic acids such as azelaic acid; retinoids such as tretionoin (synonym: all-trans-retinoic acid), all-trans-retinal and 13-cis-retinoic acid (isotretinoin); antiandrogens (5alpha-reductase) and antibiotics such as clindamycin, tetracycline and erythromycin.

Said substances usually have only a very moderate antimicrobial activity against *P. acnes* and have to be used in a relatively high concentration - benzoyl peroxide for example with up to 5 wt.-% and azelaic acid with up to 20 wt.-% - in cosmetic and dermatological formulations.

Due to the high dosage, however, the skin is extremely disadvantageous heavily overused, which is manifested particularly in very dry skin, which is often associated in part with severe skin irritation due to the strong reduction of the pH of the skin.

The effectiveness of the strong oxidizing agent such as benzoyl peroxide; the alpha hydroxy acids such as salicylic acid and lactic acid and the aliphatic dicarboxylic acids such as azelaic acid is based on the inhibition of *P. acnes.*

It is desirable to have a cosmetic or dermatological preparation to provide acne treatment that has an improved activity against *Propionibacterium acnes* (*P*. *acnes*) and improved inflammatory activity especially against inflamed comedones without severely irritating the skin.

The problem is solved by a cosmetic or dermatological composition comprising a combination of 1, 2-decanediol and willow bark extract. This preparation surprisingly shows a synergistic antibacterial effect compared to the single substances.

### Experimental Part

### Determination of MIC and MBC on Gram positive bacteria

### Aim of the test

The aim of the test was to determine the antimicrobial activity of one or more samples against a micro-organism. The values obtained must be considered characteristic of the test sample for a determined microbial species under determined test conditions.

### Description of the method

A series of test tubes was prepared containing the culture broth inoculated with the test micro-organism and the substance to be tested was introduced at decreasing scalar concentrations.

The cultures were incubated at the optimal temperature and under optimal growth conditions of the preselected micro-organism and for a sufficient period of time to demonstrate development.

The test tubes in which the microbial population developed were then determined by observing visually the turbidity that is indicative of growth. Test tubes in which the substance is present at a sufficient concentration to inhibit growth remain clear. When the nature itself of the sample prevents visual reading or if it is wished to determine whether the lack of development is due to a microbiostatic or microbicidal action, transfers are made to appropriate agar media for confirmation.

### Criteria of interpretation

The concentration of sample at which growth of the micro-organisms is inhibited is considered to be the Minimum Inhibitory Concentration (MIC), while that at which viable micro-organisms are no longer present is considered to be the Minimum Bactericidal Concentration (MBC).

### A) Evaluation of the antibacterial activity of different substances against Staphylococcus species

A test to evaluate antibacterial activity against Gram positive bacteria was conducted evaluating the following substances:
Bakuchiol, Lactoferrin, Lauric acid, Resveratrol, *Salix alba* extract.

The antibacterial activity of these substances against *Staphylococcus aureus acnes* ATCC 25923 and *Staphylococcus epidermidis* ATCC 12228 was tested according to the above-described method. The results are shown in Table 1.

**Table 1**

| | ***Staphylococcus aureus* ATCC 25923** | | ***Staphylococcus epidermidis* ATCC 12228** | |
|---|---|---|---|---|
| | **MIC** (µg/ml) | **MBC** (µg/ml) | **MIC** (µg/ml) | **MBC** (µg/ml) |
| **Bakuchiol** | 6,25 | 100 | 6,25 | 100 |
| **Lactoferrin** | >20000 | - | >20000 | - |
| **Lauric Acid** | 250 | 1000 | 500 | 1000 |
| **Resveratrol** | 1000 | >100000 | 250 | >100000 |
| **Salix Alba extract** | 30000 | 100000 | 30000 | 100000 |

Minimum Inhibition Concentration (MIC) and Minimum Bactericide Concentration (MBC) of different substances against *Staphylococcus aureus acnes* and *Staphylococcus epidermidis*

The test showed poor activity of *Salix alba* extract against Gram positive bacteria with respect to other substances tested. The highest activity against the tested *Staphylococcus* species is measured for Bakuchiol.

In a further set of experiments we tested the influence of the different substances on each other.

In order to check possible synergism of *Salix alba* extract we re-tested with the same methodology as described above a mixture of Bakuchiol and *Salix alba* extract (1:1). The result is shown in Table 2.

**Table 2**

| | ***Staphylococcus aureus* ATCC 25923** | | ***Staphylococcus epidermidis* ATCC 12228** | |
|---|---|---|---|---|
| | **MIC** (µg/ml) | **MBC** (µg/ml) | **MIC** (µg/ml) | **MBC** (µg/ml) |
| **Bakuchiol + Salix alba extract (1:1)** | 6.24 | 100 | 6.24 | 50 |

Minimum Inhibition Concentration (MIC) and Minimum Bactericide Concentration (MBC) of Bakuchiol and *Salix alba* extract against *Staphylococcus aureus acnes* and *Staphylococcus epidermidis*

Surprisingly a mixture 1:1 of Bakuchiol and *Salix alba* extract is capable to reach the same MIC and MBC of the same amount of Backuchiol if taken alone for both tested species.

These results clearly show the capability of *Salix alba* extract, which alone has only poor antibacterial activity, to improve the antibacterial activity of other substances.

### B) Evaluation of the antibacterial activity of different substances against P. acnes

In a further set of experiments we tested the antibacterial activity of *Salix alba* extract and 1,2-decanediol, a substance which is known to show antibacterial activity against *P. acnes,* alone and in combination according to the above-described method.

The microbial strain used was *Propionibacterium acnes* ATCC 11827 titrated at a concentration of 2.6 x 104 cells per millilitre of medium.

The result is shown in Table 3.

**Table 3**

| | ***Propionibacterium acnes*** | |
|---|---|---|
| | MIC (µg/ml) | MBC (µg/ml) |
| *Salix alba* extract | 1560 | 3120 |
| 1,2-decanediol | 620 | 1250 |
| *Salix alba* extract +1,2-decanediol (1:1) | 620 | 620 |
| benzoyl peroxide | 480 | 480 |

Minimum Inhibition Concentration (MIC) and Minimum Bactericide Concentration (MBC) of 1,2-Decanediol and *Salix alba* extract against *Propionibacterium acnes*

### Salix alba extract alone shows only poor antibacterial activity.

1,2-decanediol is known to be effective against *P. acnes.*

The combination (1:1) of *Salix alba* extract and 1,2-decanediol surprisingly displayed a synergistic antibacterial effect against *P. acnes.*

A combination of 1,2-decanediol and *Salix alba* extract is capable to obtain the same MIC of the double concentration of 1,2-decanediol and to improve dramatically its MBC.

The selected order degree of the association is in line with benzoyl peroxide that is commonly known as strong antibacterial agent against *P. acnes.*

Formulations can be of various nature both water-based, hydro-alcohol and anhydrous. Among the most commonly used include: surfactants systems (including even such as shampoo , bubble bath, shower gel , liquid soaps and solid soaps , micellar waters, liquid detergents), water -in-oil emulsions, oil-in-water emulsions, serums, gels, oils, aqueous solutions , hydroalcoholic solutions , anhydrous sticks, compact founding powders, lotions, milks, suncreams, after sun products and camouflage products (i.e. CC creams, BB creams).

It is optionally possible and advantageous to use the compositions according to the invention as a base for pharmaceutical formulations.

It is of course known to the person skilled in the art that cosmetic compositions are usually inconceivable without the customary auxiliaries and additives. Among these are included, for example, consistency-imparting agents, fillers, perfume, colorants, emulsifiers, additional active compounds such as vitamins or proteins, sunscreens, stabilisers, insect repellents, alcohol, water, salts, substances having proteolytic or keratolytic activity, thickeners, emollients, fatty acids, chelating agents, soothing agents, glycols, colorants, buffering substances.

The willow bark extract is prepared according to the Ph.Eur. monograph "Extracta". Whole or fragmented dried bark of young branches or whole dried pieces of current year twigs of various species of genus *Salix* including *S*. *purpurea L., S. saphnoides Vill. And S. fragilis L.* are extracted with water and/or ethanol The total content of salicylic derivatives expressed in salicin has to be at least 5%; preferably between 10 and 20%.

The *Salix alba* extract according to the present invention has to be titrated to a total concentration of salicylic derivatives expressed as salicin not less than 20 % (w/w).

The ratio between *Salix alba* extract and 1,2-decanediol is between 1:100 and 100:1, or between 1:50 and 50:1, or between 1:10 and 10:1, preferably 1:5 and 5:1; more preferably 1:1.

The overall amount of *Salix alba* extract in the formula is in a range between 0.001% and 10%, preferably between 0.01 % (w/w) and 5 % (w/w), more preferably between 0.3 % (w/w) and 1 % (w/w).

The overall amount of 1,2-decanediol in the formula is in a range between 0.001 % (w/w) and 10 % (w/w), preferably between 0.01 % (w/w) and 5 % (w/w), more preferably between 0.3 % (w/w) and 1 % (w/w).

### Examples

The following examples shall not represent a restriction of the invention.

### Example 1

### Suncream

| **INCI NAME** | **% (w/w)** |
|---|---|
| | |
| Avobenzone | 0-5 |
| Glycerin | 0-5,00 |
| Panthenol | 0-1,00 |
| Disodium EDTA | 0,10 |
| Glyceryl Stearate, PEG-100 Stearate | 1-5,00 |
| 4-Methylbenzylidene Camphor | 1-4,00 |
| Citrus Aurantius Amara | 0-1 |
| Xanthan Gum | 0-1 |
| Ascorbyl Tetraisopalmitate | 0,20 |
| Polyacrylamide, C13-14 Isoparaffin, Laureth-7 | 0-2,00 |
| Methylene Bis-Benzotriazolyl Tetramethylbutylphenol, Aqua, Decyl Glucoside, Propylene Glycol, Xanthan Gum | 0-2,00 |
| Ethylhexyl Methoxycinnamate | 0-8,00 |
| Niacinamide | 0-1 |
| Magnesium Aluminum Silicate | 0-0,50 |
| Dicaprylyl Carbonate | 1-5,00 |
| 1,2-Hexanediol (and) Caprylyl Glycol | 0,50 |
| Soy Isoflavones | 0,30 |
| Ethylhexyl Triazone | 0-2,00 |
| Titanium Dioxide,Silica, Dimethicone | 0-5 |
| 1,2-Decanediol | 0,30 |
| Salix Alba (Willow) Bark Extract | 0,30 |
| Tocopheryl Acetate | 0,50 |
| BHT | 0-0,05 |
| Hydroxyacetophenone | 0-0,50 |
| | |
| **DEM WATER** | **To 100,00** |

### Example 2

### Serum

| **INCI NAME** | **% (w/w)** |
|---|---|
| | |
| Sodium Hydroxide | 0-8 |
| Glycolic Acid | 0-20,00 |
| Lactic Acid | 0-3 |
| Niacinamide | 1,00 |
| Caprylyl Glycol | 0-5,00 |
| Xanthan Gum | 0,10 |
| Polyacrilate Crosspolymer-6 | 2,20 |
| Dicaprylyl Ether | 0-4,00 |
| Salicilic Acid | 0-2 |
| 1,2-Decanediol | 1,00 |
| Polyglyceryl-6 Stearate, Polyglyceryl-6 Behenate | 0-4 |
| Salix Alba (Willow) Bark Extract | 0,30 |
| Aluminum Starch Octenylsuccinate | 0-2,00 |
| Dipotassium Glycirrhyzinate | 0-2 |
| Sodium Phytate, Aqua, Alcohol | 0,10 |
| | |
| | |
| | |
| **Dem water** | **To 100,00** |

### Example 3

### Hydrating Cream

| **INCI NAME** | **% (w**/**w)** |
|---|---|
| | |
| Disodium EDTA | 0,10 |
| Glycerin | 0-7,00 |
| Sodium Hyaluronate | 0-1 |
| Niacinamide | 1,00 |
| Caprylyl Glycol | 0-2,00 |
| Xanthan Gum | 0,10 |
| Polyacrilate Crosspolymer-6 | 0-3,00 |
| Dicaprylyl Ether | 0-5,00 |
| 1,2-Decanediol | 0,30 |
| Polyglyceryl-6 Stearate, Polyglyceryl-6 Behenate | 2,50 |
| Salix Alba (Willow) Bark Extract | 0,3 |
| Lactic Acid | 0-1 |
| Sodium Phytate, Aqua, Alcohol | 0,05 |
| | |
| | |
| **DEM WATER** | **To 100,00** |

### Example 4

### Gel

| **INCI NAME** | **% (w/w)** |
|---|---|
| | |
| Disodium EDTA | 0,10 |
| Glycerin | 0-3,00 |
| Niacinamide | 1,00 |
| Caprylyl Glycol | 0-2,00 |
| Xanthan Gum | 0,20 |
| Polyacrilate Crosspolymer-6 | 0-3,0 |
| 1,2-Decanediol | 0,50 |
| Salix Alba (Willow) Bark Extract | 0,50 |
| Lactic Acid | 0,50 |
| Sodium Phytate, Aqua, Alcohol | 0,10 |
| | |
| | |
| **DEM WATER** | **To 100,00** |

### Example 5

### Liquid detergent

| **INCI NAME** | **% (w/w)** |
|---|---|
| | |
| Disodium EDTA | 0,10 |
| Glycerin | 0-4,00 |
| Hydrohypropyl Guar Hydroxypropyltrimonium Chloride | 0-1,0 |
| Niacinamide | 0,30 |
| Methylpropanediol, Caprylyl Glycol, Phenylpropanol | 0-5,0 |
| Xanthan Gum | 0,30 |
| Sodium Cocoamphoacetate | 0-10,0 |
| Disodium Laureth Sulfosuccinate | 0-10,0 |
| 1,2-Decanediol | 1,00 |
| PEG-40 Hydrogenated Castor Oil | 1,00 |
| Salix Alba (Willow) Bark Extract | 0,01 |
| Lactic Acid | 1,00 |
| | |
| | |
| **DEM WATER** | **TO 100,00** |

### Example 6

### CC Cream

| **INCI NAME** | **% (w/w)** |
|---|---|
| | |
| Aqua | 56,00 |
| Glycerin | 2,00 |
| Niacinamide | 0,50 |
| Salix Alba (Willow) Bark Extract | 0,50 |
| Magnesium Sulfate Heptahydrate | 0,50 |
| Methylpropanediol, Caprylyl Glycol, Phenylpropanol | 2,00 |
| Cetyl Dimethicone | 2,00 |
| Octyldodecanol | 10,00 |
| Ethylhexyl Palmitate | 8,00 |
| Dimethicone | 1,50 |
| Hydrogenated Castor Oil | 1,50 |
| 1,2-Decanediol | 1,00 |
| Cetyl PEG / PPG-10/1 Dimethicone | 2,50 |
| Titanium dioxide | 5,00 |
| Tocopheryl Acetate | 1,00 |
| Mica, Titanium Dioxide | 4,00 |
| Mica, Iron Oxides, Titanium Dioxide | 2,00 |
| | |
| **DEM WATER** | **TO 100,00** |

## Claims

1. Cosmetic or dermatological composition comprising 1, 2-decanediol and *Salix alba* extract.

2. Composition according to claim 1, **characterized in that** the proportion of 1, 2-decanediol is in the range of more than 0.1 % (w/w), In particular in the range of more than 0.2 % (w/w), based on the total mass of the preparation.

3. Composition according to one of the preceding claims, **characterized in that** the proportion of *Salix alba* extract is in the range of 0.001 % (w/w) and 10 % v, preferably between 0.01 % (w/w) and 5 % (w/w), based on the total mass of the preparation.

4. Composition according to one of the preceding claims, **characterized in that** the ratio between Salix alba extract and 1,2-Decanediol is between 1:10 and 10:1; preferably between 1:5 and 5:1, more preferably 1:1.

5. Surfactant system, water-in-oil emulsion, oil-in-water emulsion, serum, gel, oil, aqueous solution , hydroalcoholic solution , anhydrous sticks, compact founding powder, lotion, milk, suncream, after sun product or camouflage product.customary auxiliaries and additives.
